(19) Europäisches Patentamt
European Patent Office
Office européen des brevets

(11) Publication number: **0 256 566 B1**

(12) **EUROPEAN PATENT SPECIFICATION**

(45) Date of publication of patent specification: **06.11.91**

(51) Int. Cl.$^5$: **A61K 7/26**, A61K 7/16

(21) Application number: **87201124.2**

(22) Date of filing: **12.06.87**

# (54) Use of usnic acid or derivatives thereof in the treatment of dental caries.

(30) Priority: **25.06.86 IT 2090286**

(43) Date of publication of application:
**24.02.88 Bulletin 88/08**

(45) Publication of the grant of the patent:
**06.11.91 Bulletin 91/45**

(84) Designated Contracting States:
**AT BE CH DE ES FR GB GR IT LI LU NL SE**

(56) References cited:
**FR-A- 2 081 338**
**US-A- 4 139 609**

**PATENT ABSTRACTS OF JAPAN, vol. 6, no. 49 (C-96)[927], 31st March 1982; & JP-A-56 666 111 (LION K.K.) 21-12-1981**

**MANUFACTURING CHEMIST, vol. 57, no. 5, May 1986, London, GB; "Natural products with antibacterial activity and surface active properties on the increase"**

**CHEMICAL ABSTRACTS, vol. 82, 1975, page 292, abstract no. 47615n, Columbus, Ohio, US; M. FONTANA et al.: "Usnic acid as natural preservative deodorant, and antimicrobial agent in cosmetic systems", & RIV. ITAL. ESSENZE, PROFUMI, PIANTE OFF., AROMI, SAPONI, COSMET., AEROSOL 1974, 56(6), 315-36**

**CHEMICAL ABSTRACTS, vol. 72, 1970, page 232, abstract no. 103746p, Columbus, Ohio, US; & JP-A-70 02 749 (EISAI CO., LTD) 29-01-1970**

(73) Proprietor: **ISCOFAR Sas di Paolo E. Ghirardi**
**Corso Venezia 16**
**I-20121 Milano(IT)**

(72) Inventor: **Ferrari, Giorgio**
**Via Borgonuovo 7**
**I-20100 Milano(IT)**
Inventor: **Ghione, Mario**
**Via Assietta 31**
**I-20100 Milano(IT)**
Inventor: **Ghirardi, Paolo**
**Via Podgora 1**
**I-20100 Milano(IT)**

(74) Representative: **Dragotti, Gianfranco et al**
**SAIC BREVETTI s.r.l. Viale Bianca Maria, 15**
**I-20122 Milano(IT)**

**EP 0 256 566 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to compositions containing usnic acid or its derivatives useful for the therapeutical control of caries, particularly for the preventive treatment and for the therapy of the cariogenic dental plaque.

It is known that the primary pathogenic microorganisn of the cariogenic lesions and thus of the pathogenesis of the dental caries is the Streptococcus mutans.

Studies carried out since several years about the pathology and pathogenis of dental caries and of the periodontal diseases, studies which were very intense taking it into account the social incidence of these pathologies, permitted the rule of this bacterium to be clarified: it, as some other bacterial species of saccharolitic nature, it capable of adhering to smooth surfaces, such as the dental enamel coated by a saliva layer, forming great viscous structures, consisting of glucose polymers, which in a short time (of the order of hours) become the seat of bacterial colonies.

In this way the bacterial plaque is formed , under which and under the protection of which the cariogenic activity prosecutes in practically not disturbed manner.

The Streptoccocus mutans is sensitive towards several antibiotics even at rather low concentrations, but the efficacy of these bactericide agents for the prophylaxis and the treatment of the cariogenic plaque is very limited (see Fitzgeral RJ, Inhibition of experimental dental caries by antibiotics, Antimicrob. Agents. Chemoth., 1, 296, 1972; De Paol PR, Jordan AV, Berg J. Temporary suppression of S.mutans in humans through topical application of Vancomycin, J. Dent. Res. 53, 108, 1974).

As a matter of fact, paradoxically, the inhibitors so far tested not only increase the adherence of Streptococcus mutans (see Ghione M;, Meixelsperger G., Pelizzoni G., Gliozzi T., Influence of subinhibitory concentrations of antibiotics on aggregation and adherence of Streptococcus mutans. In: The influence of antibiotics on the host-parasite relationship II. Adam D., Hahn H., Opferkuch W., Eds. Springer Verlag, Berlin Heildelberg, p. 209, 1985), but also alter the microbiologic equilibrium of the oral cavity thus becoming a side cause of iatrogenic stomatitis. According to further more recent research work, inhibitors which are specifically or preferentially active against Streptococcus mutans have been searched and tested, these attempts being based on the use of antibodies (Furuta T., Nisizawa T., Chiba J., Hamada S., Production of monoclonal antibody against a glucosyltransferase of Streptococcus mutans 6715, Infect. Imnun., 41, 822, 1983); enzymes (Olami Y., Takashio M., Umezawa H., Ribocitin, a new inhibitor of dextran sucrose, J. Antib., 34, 344, 1981; Endo A., Hayashida O., Murakawa S. Mutastein, a new inhibitor of adhesive insoluble glucan synthesis by glucosyltransferase of Streptococcus mutans, J. Antib., 36, 203, 1983); and antibiotics (see Pallanza R., Scotti R., Beretta G., Cavalieri B., Arioli V. In Vitro activity of A-16686 a potential antiplague agent, Antimicrob. Agent. Chemother., 26, 462, 1984).

The failure of these attempts as a matter of fact seems to be attributable to the peculiar behaviour of Streptococcus mutans which, under the action of known antibiotics with wide spectrum at concentration even less than the minimum inhibiting concentrations, from the normal spherical shape, having rigid cellular walls, assume a flattened and flappy shape, whereby it clings even more tenaciously to the tooth surface, and thus the dental bacterial plaque is also more tenacious.

It is furthermore to be added that the local treatment, namely in the oral cavity, with antibiotics may give place to objectionable side effects, which are particularly serious in patients having reduced immunitary defences, as the development of bacterial species resistant to antibiotics.

It has been now found and is the main object of the present invention that the forming of the dental plaque can be efficaciously prevented and the elimination thereof can be readily achieved, thus radically solving the problems and drawbacks as above shortly mentioned, by having recourse to a local treatment of the teeth and of the oral cavity with usnic acid or derivatives thereof.

The usnic acid is a natural substance known since long time which is found in several plants and especially in some lichens.

The chemical structure thereof corresponds to 4,8-diacetyl-3,7-dihydroxy-2,9a-dimethyl-9-oxo-9H-dibenzofurane and to the formula:

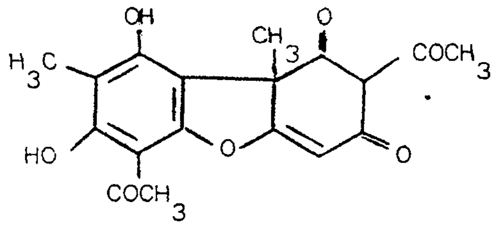

The usnic acid is found, in nature, in both the optically active forms (d) and (l) , and as racemic mixture.

For the usnic acid in the literature several biological activities are reported: as antibacterial and antitumoral substance (C.A. 91: 16818 c; C.A. 93; 179563f; C.A. 80; P 306952z; C.A. 93: 88256s; C.A. 121096f); as antispatic and antihistaminic (C.A. 91: 13609j); as antiinflammatory (C.A. 77: 111574z; C.A. 102: 209128 s) and as local anaesthetic (C.A. 95: 197518r).

However the specific activity of this antibiotic against the Streptococcus mutans not only has never been disclosed, but nothing might even suggest that usnic acid could have a so selective activity with respect to such a bacterium, as it has been demonstrated from the in vitro and in vivo tests as reported hereinafter.

As a confirmation a paper can be cited (Fontana M. et al. Riv. Ital., Essenze, Profumi, Piante Offic., Aromi, Saponi, Cosmet., Aerosol, 1974, 56(6), pages 315-336) which is just dealing with the usnic acid and its use in cosmetic formulations among which also tooth pastes and tooth gels are cited: in the paper the results of tests of antibacterial activity with respect to a number of common bacterial species is reported in order to show the preserving and disinfecting activity, but without even a word about Streptococcus mutans or at least about the bacterial dental plaque. On the contrary, just in the case of the use as tooth paste, it is foreseen that other substances having a specific antibacterial and disinfecting activity are combined with usnic acid.

It was therefore fully unexpected and surprising to find that usnic acid has a relevant and specific bacteriological activity with respect to the Streptococcus mutans, which, as already said, from the laboratory and clinical esperiments seems to be the primary pathogenic microorganism of the dental caries. A feature which is surprising as well and more specifical of the present invention is that the usnic acid is selectively active against the Streptococcus mutans whereas it is practically without activity towards the other bacteria of the oral cavity. A further feature of the present invention is that the dextorotatory form of the usnic acid is sensibly more active than the levorotatory form.

In this connection and as a confirmation, in the table 1 the activity spectrum found for the (+) usnic acid and for (-) usnic acid is reported, with respect to a certain number of pathogenic microorganisms.

TABLE 1

| Strain | (+) usnic acid (DI) | (-) usnic acid (DI) |
|---|---|---|
| C. Albicans B.1003 (1) | > 100 | > 100 |
| C. Parapsylopsis LCC 1008 (1) | > 100 | > 100 |
| C. krusei (1) | > 100 | > 100 |
| C. pseudotropicalis ATCC 9667 (1) | > 100 | > 100 |
| Streptococcus mutans (2) | 10 | 100 |
| Staphylococcus aureus Cowsn I (2) | 100 | > 100 |
| Streptococcus milleri (2) | 30 | 50 |
| Klebsiella aerogenes 1082 E (3) | > 100 | > 100 |
| Pseudomonas fluorescens 2479 (3) | > 100 | > 100 |
| Escherichia coli K 12 (3) | > 100 | > 100 |
| Salmonella sp. 11 (3) | > 100 | > 100 |
| The DI (minimum inhibiting dose) are expressed in µg/ml<br>(1) = Candida; (2) = Gram (+); (3) = Gram (-). | | |

The tests have been carried out in Sabouraud medium agarized for the yeast colture and in a Müller-Hinton medium, again agarized for the bacterial colture. The sterile usnic acids were dissolved in amounts of 20 mg in sterile acetone. By means of subsequent acetonic dilutions and by adding the solution to the

above reported media, melted at 60° C a series of medicated plates was obtained at the final concentrations of 100 µg/ml, 10 µg/ml, 1 µg/ml; 0.1 µg/ml and 0.01 µg/ml.

The collection strains used for the tests were taken from the deep-freeze and subcultivated for two passages, in liquid Sabouraud medium per the Candidae and in BHIB medium (Brain Heart Infusion Broth) for the bacteria. The medicated plates, inoculated with a microdrop, have been incubated at 37° C. After 48 hours the growth was evaluated.

According to another feature of the present invention an especially significant synergic activity is obtained with equimolar amounts of usnic acid and hexetidine. The activity data of such a composition are reported in Table 2.

TABLE 2

Sensibility expressed as minimum inhibiting dose in mcg/ml of some strains of Streptococcus mutans against (+) usnic acid (AU), hexetidine (HE) and equimolar compositions of the two substances (CE)

| Strains of Streptococcus mutans | AU | HE | CE |
|---|---|---|---|
| MI 27 | 20 | 20 | 5 + 5 |
| C 3,26 | 20 | 30 | 5 + 5 |
| M 34 | 5 | 10 | 1 + 1 |
| MI 50 | 5 | 5 | 1 + 1 |
| 125 | 20 | 25 | 5 + 5 |
| M 2.35 | 20 | 30 | 10 + 10 |
| SMI | 15 | 20 | 5 + 5 |

The tests have been carried out in BHIB (Brain heart Infusions Broth) contained in sterile test tubes and medicated at final concentrations of 100-50-30-25-20-15-10-5-1-0.5 µg/ml of the substances being tested or of their equimolar mixture.

The used strains of Streptococcus mutans are isolated strains, previously typized and characterized.

Those strains have been taken from the deep-freezer (-80° C) and subcultivated in BHIB medium for 24 hours at 37° C in anaerobic atmosphere. Then they have been seeded in test tubes containing 5 ml of BHIB and the solutions of the composition of usnic acid and hexitedine in amounts of 0.2 ml have been put in incubation under the conditions of the subculture. The solutions of the composition of usnic acid and hexetidine have been prepared by dissolving the usnic acid in amount of 10 mg in 2 ml of acetone and adding to such a solution an equivalent amount of hexetidine, the latter being also dissolved in 2 ml of acetone.

Then the solution has been then suitably divided out and diluted in order to obtain the above indicated concentrations.

Hexetidine may be found in 3 forms: the mesoform and the two active forms.

The present invention comprises the composition containing usnic acid and hexetidine both as single isomer and as mixture of its isomers.

According to a further aspect of the invention the bacteriostatic activity of usnic acid against Streptococcus mutans may be enhanced by the presence of suitable amounts of natural antibiotics, such as for instance tetracyclines and their derivatives such as pyrrolidinomethyltetracycline , 6-thiotetracycline; fortimycin B; aminocyclitols, fusidic acid, avernic acid, clindamycin, lincomycin; spiramycin; cephaloridine. The activity is also increased by the presence of sinthetic antimicrobials such as chlorexetidine; 3,4-dihydroxyphenethylic alcohol; sulfamidic compounds; sanguinarine.

Compositions containing usnic acid to be used for the oral and tooth igiene or in the medications for dentists, for the prevention and/or the treatment of dental caries according to the present invention comprise: tooth pastes, tooth powders, liquid dentifrices, non-soap dentifrices, mouth washes , products for hygiene of dental prostheses, such as liquid and powder products for the cleaning of dental plates,

medicated preparations for dentists such as antiseptic solutions, tinctures, medicated materials for the care and filling of teeth and cavities, and for stoppings, pastes for mummifications of dental pulp, anodyne medications, varnishes, sterilized solutions, varnishes for cavities, waxes, resins, creams, gels, etc. Moreover the compositions of the present invention may be incorporated in special preparations for the prevention of caries such as chewing-gum, pearls, lozenges, tablets and medicated gums, including the coarcevates or other preparations for intradental use even with delayed action. The liquid preparations may also be formulated in the spray forms.

In the above listed compositions the usnic acid may be introduced in doses of between 5 µg and 100 mg per 1 ml or 1 g of preparate. The usnic acid present in the preparates may consist of pure acid in the single optically active forms, or of the racemic mixture as micronized powder; alternatively it may be in form of extract or powder of plants containing it.

The usnic acid may also be used in form of compounds such as esters and/or acyl derivatives or soluble salts, such as for example sodium salt; compounds with alkali phosphates and hexametaphosphates; salt with lysozyme; salt with thrometamine; addition compounds with aminosugars, compounds with triethanolamine with aminoethyl propandiole and others. Since the usnic acid is a compound having the behaviour of a mono basic acid, other bases suitable from the pharmaceutical point of view, besides the mentioned one, may be used to obtain suitable compounds for the use thereof in the hygienic-antiseptic preparation, such as for example diethylamine, ethylamine, triethylendiamine, morfoline, piperazine, piperidine, guanidine, pyperidinol, betaine, litium and potassium hydroxide and carbonate.

Likewise suitable are the basic aminoacids such as lysine and arginine. Particolarly suitable is the use of equimolar amounts of (+) usnic acid and L-lysine.

In order to obtain high concentrations of the derivatives of usnic acid in the formulations it has been found useful to use aprotic bipolar solvents acceptable from the pharmaceutical point of view. It has been particularly found that especially suitable to obtain a high solubility are dimethylacetamide, diethylacetamide, tetramethylurea, and N-methylpyrrolidone.

The compositions containing usnic acid or derivatives thereof, as above, are provided according known techniques with the use of solvents , excipients, auxiliaries, stabilizers, anti-oxidants, preservants, US-rays absorbing substances, suiteners, flavoring, perfumes, surface active agents, lubricants, drying agents, etc. of current use in the pharmaceutical and hygienic-antiseptic preparations.

As already mentioned the compositions of the present invention have been subjected to pharmacological tests and to in vivo experiments in volunteers.

The antibacterial activity of semples of D(+) and L(-) usnic acid, and other antibiotics has been tested on strains of Streptococcus mutans freshly isolated from human dental lesions as well as ATCC 25175 strain growing in either brain heart infusion broth (BHIH Difco) or chemically defined medium (CDM). CDM contains sorbitol, amino-acids, purines and pyrimidine bases, vitamins, fenol red as indicator and was sterilized by filtration.

Minimal inhibitory concentration (MIC) values were ascertained on the basis of visual or turbidimetric reading.

Adherence tests in uncoated or salive coated microtiter plastic walls were carried out as described by Ghione M. et al (cit. loc.)

In vitro tests were carried out in 10 informed and consenting volunteers by sampling bacterial flora of tooth surface before and after application of usnic acid preparations.

a) In vitro

Microbiological tests confirmed time honored knowledge on the preponderant activity of usnic acid against Gram-positive bacteria and supplied a new piece of information by making manifest the higher activity of the dexotrorotatory (+) form and the peculiar sensitivity of Streptococcus mutans strains.

No consistent difference in turbidimetric visual or strumental reading was observed to occur between tests carried out in aerobic BHIB or anaerobic CDM cultures.

Tests based on the appreciation of indicator color change as growth marker in CDM cultures in plastic wells generated the data given in table 3.

TABLE 3

| | MIC µg/ml | |
|---|---|---|
| | C | U |
| Usnic Acid | 2.8 (2.4-3) | 2.8 (2.4-3) |
| Clindamycin | 3.2 (1-12) | 4.0 (2-9) |
| Lincomycin | 0.45 (0.18-0.7) | 0.60 (0.3-1.4) |
| Spiramycin | 1.8 (0.5-2.5) | 4.0 (3-6) |
| Erythromycin | 0.9 (0.5-2) | 1.2 (0.6-3) |
| Cephaloridin | 0.01 (0.005-0.1) | 0.006 (0.003-0.1) |
| Cephazolin | 0.3 (0.2-0.4) | 0.3 (0.2-0.4) |
| Cephtizoxime | 0.5 (0.04-0.7) | 0.5 (0.3-0.7) |
| Cephyroxime | 0.2 (0.1-0.5) | 0.2 (0.1-0.5) |

Average values from tests carried out in uncoated (U) or salive coated (C) plastic wells.

Difference in MIC values between Table 1 and Table 3 data are due to difference in parameters taken as index of bacterial growth.

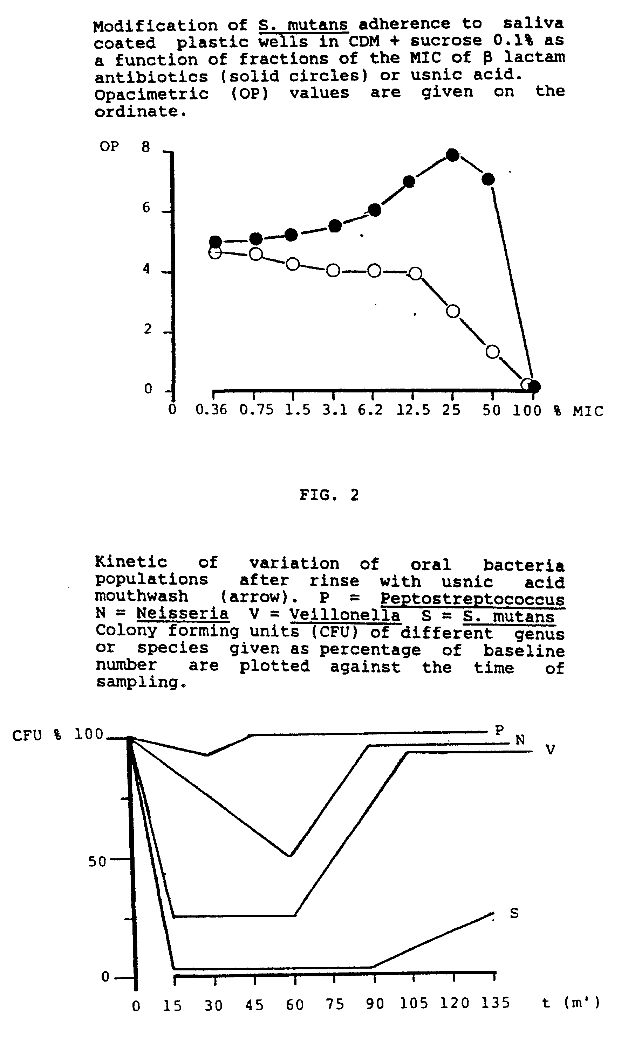

Table 3 data show that in absolute terms the growth inhibiting activity of the majority of tested antibiotics was higher than that of usnic acid, but for a correct evaluation of the difference existing between usnic acid and other antibiotics, as far as the activity of Streptococcus mutans is concerned, not only quantitative but also qualitative criteria shall be taken into consideration. Indeed the growth of Streptococcus mutans in the presence of subinhibitory concentrations of usnic acid was not accompanied by increase in adherence to smooth surfance at variance with what observed with other antibiotics. The different behaviour is show in fig. 1.

b) In vivo

Oral rinse with mouthwash preparations containing usnic acid (as lysine salt) was observed to induce a peculiar alteration of the oral bacterial flora. The time course of the phenomenon is given in fig. 2.

The fiture shows that quick sharp and long lasting decrease in number of Streptococcus mutans CFR, together with limited derangement of other bacterial genera, were induced by oral rinse with usnic acid.

In order to illustrate by without limiting purposes the present invention the following examples are reported relating to the preparation of compositions to be used for the preventing treatment or for the care of the dental plaque and consequently for the therapeutical control of the caries.

| EXAMPLE 1 mouth wash | |
|---|---|
| (+) usnic acid | g 10 |
| Hexetidine | g 10 |
| Dimethylacetamide | ml 100 |
| Polysorbate 80 | ml 16.5 |
| Glycerin | ml 200 |
| Alcohol 95° | ml 600 |
| Sodium saccharin | g 0.5 |
| mint oil | q.s. |
| Water | q.s. to ml 1000 |

The (+) usnic acid is suspended in dimethylacetamide, is heated in water bath at 50° C, and under stirring the hexetidine is gradually added. The alkalinity of the solution is adjusted to pH 8 by adding hexetidine as needed.

Then polysorbate, half of the alcohol, glycerin, the other half of the alcohol and subsequently the other components of the recipe are added, the sodium saccharine having been previously dissolved in water, always under stirring.

The mixture is filtered and distributed in small glass bottles with dropper e.g. having a content of 20 ml.

Each ml of the thus obtained solution contains 10 mg of usnic acid. The solution, for the use, is diluted with water for example 20 drops in 15 ml of water for mouth, washing. The mouth wash can be used also in

the appliance for dental shower: 20/40 drops, or more, diluted in the water filling the tank of the appliance.

EXAMPLE 2

The example 1 is repeated, by substituting an equivalent amount of tetramethylurea for the dimethylacetamide.

EXAMPLE 3

Example 1 is repeated by substituting an equimolar amount of L(+) lysine (g 4.4) for the hexetidine.

| EXAMPLE 4 Tooth paste | |
|---|---|
| (+) usnic acid | mg 250 |
| sodium monofluorophosphate | mg 200 |
| di-calcium phosphate | g 50 |
| silica | g 50 |
| cellulose gum | g 50 |
| glycerin | g 25 |
| vaseline oil | g 10 |
| sodium laurilsulphate | g 5 |
| methylparaben | mg 250 |
| propylparaben | mg 250 |
| sodium cyclamate | mg 200 |
| sodium saccharin | mg 50 |
| flavors | q.s. |
| ethylcellosolve | ml 80 |
| water | to g 1000 |

The usnic acid is dissolved in 80 ml of ethylcellosolve (ethyleneglycol monoethylether) by heating to 50 °C.

The cellulose gum and silica are hydrated by using 500 ml of water heated to 35-40 °C.

The sodium monofluorophosphate, the sodium cyclamate and the sodium saccharine are dissolved in 200 ml of water; this solution is added with methylparaben and propylparaben in the indicated amount and previously dissolved in 10 ml of 95° alcohol . The glycerin is diluted in the remaining amount of water. The dicalcium phosphate, mixed together with laurilsulphate, is added to the ingredients previously introduced in the mixer.

The mixture is worked until a homogeneous and uniform paste is obtained. Then the vaseline oil is added , it having being supplemented with the desired flavors, and the paste is further kneaded up to homogeneous incorporation. Lastly the product is packaged in small pipes of the desired weight.

Example 5

Example 4 is repeated by substituting for the (+) usnic acid an equivalent amount of titred extract obtained from Usnea dasupoga (Ach.) Nyl (usneaceae).

Example 6

Example 4 is repeated by substituting for (+) usnic acid 500 mg of combined (+ usnic) acid/ hexetidine 1:1 as prepared in tetramethylurea solution.

| EXAMPLE 7 Tooth paste | |
|---|---|
| Sorbitol | g 50 |
| cellulose gum | g 50 |
| silica | g 30 |
| micronized kaolin | g 25 |
| mica | g 10 |
| sodium laurilsulphate | mg 150 |
| (+) usnic acid | mg 250 |
| sodium monofluorophosphate | mg 150 |
| sodium fluoride | mg 100 |
| sodium saccharie | mg 350 |
| sodium hydroxide N | ml 9 |
| metilparaben | mg 250 |
| propylparaben | mg 250 |
| flavors | q.s. |
| vaseline oil | ml 10 |
| water | q.s. to g 1000 |

The sorbitol, mica, micronized kaolin and sodium laurilsulphate are mixed up to homogeneous condition. The cellulose gum and the silica are hydrated with 500 ml of water.

The usnic acid is added to the solution of sodium hydroxide. The sodium monofluorophosphate, the sodium fluoride and sodium saccharin are dissolved in 200 ml of water; this solution is added with methylparaben and propylparaben previously dissolved in 10 ml of 95° alcohol Subsequently in the mixing machine the previous ingredients are added with the remaining amount of water and the mixture is kneaded until a homogeneous mass is obtained. The desired flavors are added in vaseline oil. The mass is still kneaded up to homogeneous incorporation and then small pipes of the desired weight are packaged.

EXAMPLE 8

The example 7 is repeated by substituting form (+) usnic acid an equivalent amount of equimolar composition of (+) usnic acid and L-lysine prepared in tetramethylurea solution. The sodium hydroxide is reduced to the amount of 1 ml.

| EXAMPLE 9 Tooth powder | |
|---|---|
| Precipitated calcium carbonate | g 470 |
| sorbitol | g 180 |
| silica | g 120 |
| colloidal kaolin | g 100 |
| dicalcium phosphate | g 100 |
| sodium laurilsulphate | g 10 |
| sodium saccharin | g 0.5 |
| (+) usnic acid | g 0.5 |
| methylparaben | g 0.25 |
| propylparaben | g 0.25 |
| flavors | q.s. |
| dye | q.s. |

In a mixer for powders all the components previously brought to a uniform size are introduced apart from flavors and usnic acid. The flavors are added with micronized (+) usnic acid micronized and the mixture is added to the rest of the powders in the mixer.

The mass is mixed up to homogeneity and uniform distribution.

Packages of the unit content as desired are made.

| EXAMPLE 10 Prepared for the hygien of artificial prostheses. | |
|---|---|
| Sodium bicarbonate | g 70 |
| Sodium perborate | g 15 |
| Sodium laurylsulphate | g 1 |
| (+) usnic acid | g 0.3 |
| L-lysine | g 0.135 |
| tetramethylurea | g 5 |
| polyethylene glycol 400 | g 7 |
| flavors | q.s. |

The sodium bicarbonate, sodium perborate and sodium laurylsulphate are ground to the same size. With (+) usnic acid and L-lysine the solution in tetramethylurea is prepared and it is added with polyethylene glicol ; the new solution is added with the powder mixture, also the flavors being added. For the use a tea spoon of the preparate is dissolved in a water glass.

| EXAMPLE 11 Chewing gum | |
|---|---|
| (+) usnic acid | mg 500 |
| sorbitol | g 1200 |
| mannite | g 1200 |
| base gum | g 580 |
| sodium saccharin | g 2.5 |
| glycerin | g 15 |
| flavors | q.s. |
| dyess | q.s. |

To the sorbitol, melted by heating to 100° C there are added under stirring, gradually, mannite and the sodium saccharin. In the sugar melted mass the base gum and the glycerin are subsequently added by kneading until the mass is consistent and plastic.

The paste is then added with the solution of (+) usnic acid in the oil or in the mixture of desired essential oils such as for instance bisazolol, representing the flavor, and the dye.

The essential oils are used in amounts of 4-8 g per each 500 g of final product. The mixture is kneaded furthermore up to homogeneous incorporation, flattened as a sheet with a roll and then cut into stripes or a desired , having a unitary weight of 3 g. The essential oils used can be for instance: mint, gaulteria, sassafrass, clove, allmond, vanilla, oris etc.

EXAMPLE 12

Example 11 is repeated by using for the (+) usnic acid an equivalent amount of equimolar combination of (+) usnic acid and L-lysine in tetramethylurea. Also 3 g of potassium glycirhizzinate are added.

| EXAMPLE 13 Paste for the root channels | |
|---|---|
| Cresol | g 12.00 |
| Eugenol | g 4.00 |
| (+) usnic acid | g 0.50 |
| L-lysine | g 0.22 |
| zinc sulphate | g 8.00 |
| zinc oxide | g 32.00 |
| tetramethylurea | g 5.00 |
| glycerin | g 38.50 |

Cresol and eugenol are mixed and the mixture is added with the solution of (+) usnic acid prepared with the L-lysine in the tetramethylurea and the same amount of glycerine.

The thus obtained solution is kneaded with the zinc sulphate and zinc oxide using the remaining glycerin , until a paste of the desired consistenc is obtained.

| EXAMPLE 14 Varnish for cavities | |
|---|---|
| copal gum<br>(+) usnic acid<br>acetone | g 60<br>g 0.5<br>ml 100 |

The (+) usnic acid is dissolved into the acetone in water bath at 40-50 °C.
The solution is added with the copal gum, stirred up to dissolution and then filtered.

## Claims

1. Use of usnic acid for the preparation of compositions for oral use suitable for the therapeutical control of dental caries and for the preventive treatment and for the therapy of cariogenic dental plaque.

2. Use of usnic acid according to claim 1, characterized in that said acid is in the optically active dextrorotatory form.

3. Composition for oral use suitable for the therapeutical control of dental caries and for the preventive treatment and for the therapy of the dental plaques, characterized by containing as the active ingredient usnic acid in the optically active dextrorotatory form together with the excipients, solvents and additives currently used in oral pharmaceutical hygienic-sanitary preparation.

4. Composition according to claim 3, characterized in that said usnic acid is contained in an amount of between 0,005 and 100 mg per ml or per gram of preparation.

5. Composition according to claim 3, characterized in that the (+) usnic acid is associated to equimolar amounts of hexetidine .

## Revendications

1. Utilisation d'acide usnique pour la préparation de compositions pour usage oral indiqué pour le contrôle thérapeutique de caries dentales ainsi que pour le traitement préventif et pour la thérapie de plâques dentales cariogéniques.

2. Utilisation d'acide usnique, selon la revendication 1, caractérisé du fait que ledit acide existe dans la forme dextrorotaroire optiquement active.

3. Composition pour l'utilisation orale indiquée pour le contrôle thérapeutique de caries dentales ainsi que pour le traitement préventif et pour la thérapie de plâques dentales, caractérisée du fait qu'elle contient comme ingrédient actif de l'acide usnique dans la forme dextrorotatoire optiquement active avec les excépients, solvants et additives couramment usés dans une préparation pharmaceutique orale hygiénique-sanitaire.

4. Composition selon la revendication 3, caractérisée du fait que ledit acide usnique est contenu dans une quantité entre 0,005 et 100 mg par ml ou par gramme de préparation.

5. Composition selon la revendication 3, caractérisée du fait que l'acide usnique (+) est associé à des quantités d'hexétidine équimoleculaires.

## Patentansprüche

1. Verwendung von Usninsäure zur Zubereitung von Zusammensetzungen zum Einnehmen, geeignet zur therapeutischen Kontrolle von Zahnfäule sowie zur Vorbehandlung und zur Therapie von kariogenischem Zahnbelag.

2. Verwendung von Usninsäure, gemäß Anspruch 1, dadurch gekennzeichnet, daß besagte Säure in optisch aktiver Dextrorotatorform besteht.

3. Zusammensetzung zum Einnehmen, geeignet zur therapeutischen Kontrolle von Zahnfäule sowie zur Vorbehandlung und zur Therapie von Zahnbelag, dadurch gekennzeichnet, daß sie als aktiver Bestandteil Usninsäure in optisch aktiver Dextrorotatorform enthält, zusammen mit Füllmitteln, Lösungen und Zusatzstoffen, die bei einer pharmazeutischen hygienisch-sanitären Einnahmezubereitung laufend verwendet werden.

4. Zusammensetzung, gemäß Anspruch 3, dadurch gekennzeichnet, daß besagte Usninsäure in einer Menge zwischen 0,005 und 100 mg pro Milliliter oder pro Gramm der Zubereitung enthalten ist.

5. Zusammensetzung, gemäß Anspruch 3, dadurch gekennzeichnet, daß die (+) Usninsäure mit gleichen Molekülärmengen von Hexetidin assoziiert ist.

Modification of S. mutans adherence to saliva coated plastic wells in CDM + sucrose 0.1% as a function of fractions of the MIC of β lactam antibiotics (solid circles) or usnic acid. Opacimetric (OP) values are given on the ordinate.

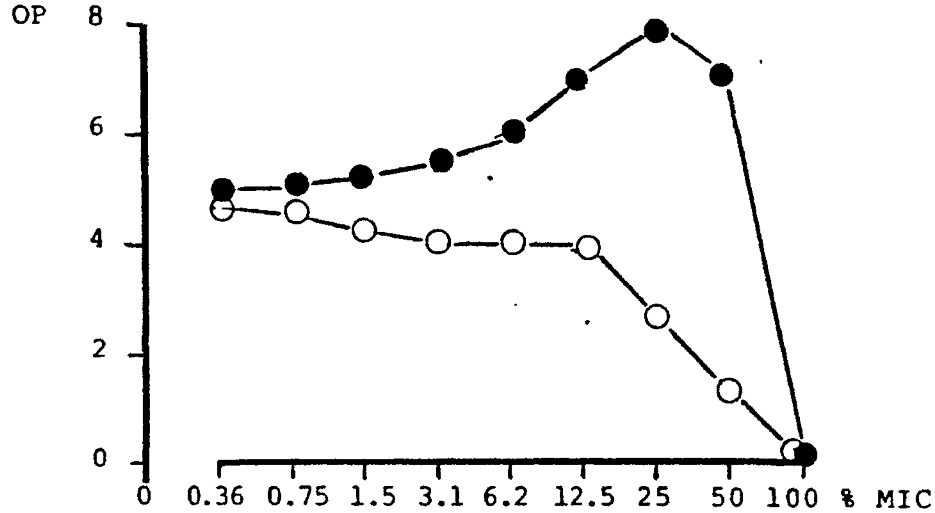


FIG. 2

Kinetic of variation of oral bacteria populations after rinse with usnic acid mouthwash (arrow). P = Peptostreptococcus N = Neisseria V = Veillonella S = S. mutans Colony forming units (CFU) of different genus or species given as percentage of baseline number are plotted against the time of sampling.